(19) Europäisches Patentamt
European Patent Office
Office européen des brevets

(11) Veröffentlichungsnummer : **0 144 033**
**B1**

(12) # EUROPÄISCHE PATENTSCHRIFT

(45) Veröffentlichungstag der Patentschrift : **20.01.88**

(21) Anmeldenummer : **84113928.0**

(22) Anmeldetag : **17.11.84**

(51) Int. Cl.[4] : **C 07 C 49/163**, C 07 C 49/167, C 07 C 45/62, C 07 C 49/23, C 07 C 49/235, C 07 C 45/74, C 07 D249/08, C 07 C 49/233

(54) **Substituierte 5-cycloalkyl-2,2-dimethyl-pentan-3-one.**

(30) Priorität : **01.12.83 DE 3343532**

(43) Veröffentlichungstag der Anmeldung : **12.06.85 Patentblatt 85/24**

(45) Bekanntmachung des Hinweises auf die Patenterteilung : **20.01.88 Patentblatt 88/03**

(84) Benannte Vertragsstaaten : **BE CH DE FR GB IT LI NL**

(56) Entgegenhaltungen : **EP-A- 0 095 047**
**FR-A- 2 253 505**

(73) Patentinhaber : **BAYER AG**
**Konzernverwaltung RP Patentabteilung**
**D-5090 Leverkusen 1 Bayerwerk (DE)**

(72) Erfinder : **Ziemann, Heinz, Dr.**
**Am Wiesenberg 10**
**D-5653 Leichlingen 1 (DE)**
Erfinder : **Mohrmann, Karl-Heinrich, Dr.**
**Roonstrasse 61**
**D-5600 Wuppertal 1 (DE)**

**EP 0 144 033 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue substituierte 5-Cycloalkyl-2,2-dimethyl-pentan-3-one, ein Verfahren zu deren Herstellung und ihre Verwendung als Zwischenprodukte zur Synthese von Stoffen mit pflanzenwachstumsregulierender und fungizider Wirksamkeit.

Es ist bereits bekannt geworden, daß sich Azolyl-methylketone als Zwischenprodukte zur Herstellung von Azolyl-Derivaten mit pflanzenwuchsregulierenden und fungiziden Eigenschaften verwenden lassen (vgl. EP-B 0 032 200 und EP-B 0 031 911). So kann z. B. durch Umsetzung von 1-(1,2,4-Triazol-1-yl)-3,3-bis-fluormethyl-butan-2-on mit Cyclohexyl-methyl-bromid das 1-Cyclohexyl-2-(1,2,4-triazol-1-yl)-4,4-bis-fluormethyl-pentan-3-on nach folgendem Schema synthetisiert werden :

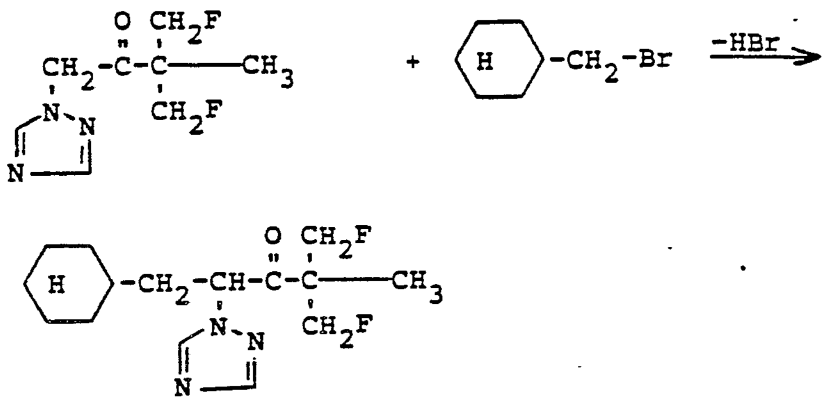


Nachteilig ist jedoch, daß bei dieser Art der Herstellung von Azolyl-Derivaten mit pflanzenwachstumsregulierender und fungizider Wirksamkeit die als Zwischenprodukte benötigten Azolyl-methyl-ketone nur durch mehrstufige Synthesen erhältlich sind und die dabei als Ausgangsstoffe eingesetzten Materialien zum Teil nur schwierig zugänglich sind.

Es wurden nun neue substituierte 5-Cycloalkyl-2,2-dimethyl-pentan-3-one der Formel (I)

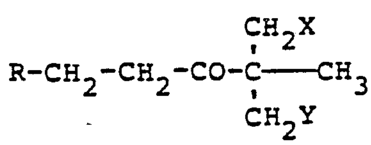
(I)

in welcher

R für gegebenenfalls einfach bis dreifach, gleich oder verschieden durch Alkyl mit 1 bis 3 Kohlenstoffatomen substituiertes Cycloalkyl mit 5 bis 7 Kohlenstoffatomen steht,

X für Fluor oder Chlor steht und

Y für Wasserstoff, Fluor oder Chlor steht,

gefunden.

Weiterhin wurde gefunden, daß man die neuen substituierten 5-Cycloalkyl-2,2-dimethyl-pentan-3-one der Formel (I) erhält, wenn man Ketone der Formeln (IIa, IIb oder IIc),

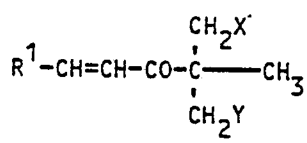
(IIa)

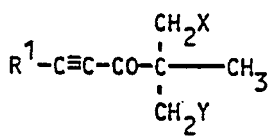
(IIb)

oder

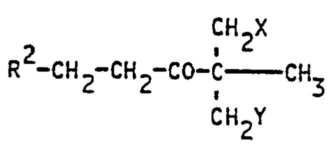
(IIc)

in welchen

X und Y die oben angegebenen Bedeutungen haben,

$R^1$ für jeweils gegebenenfalls einfach bis dreifach, gleich oder verschieden durch Alkyl mit 1 bis 3 Kohlenstoffatomen substituiertes Cycloalkyl mit 5 bis 7 Kohlenstoffatomen, Cycloalkenyl mit 5 bis 7 Kohlenstoffatomen oder Phenyl steht und

$R^2$ für gegebenenfalls einfach bis dreifach, gleich oder verschieden durch Alkyl mit 1 bis 3 Kohlenstoffatomen substituiertes Phenyl steht,

selektiv mit Wasserstoff in Gegenwart eines Hydrierkatalysators sowie gegebenenfalls in Gegenwart eines Verdünnungsmittels umsetzt.

Schließlich wurde gefunden, daß die neuen substituierten 5-Cycloalkyl-2,2-dimethyl-pentan-3-one der Formel (I) sehr gut geeignet sind als Zwischenprodukte zur Herstellung von 4-Azolyl-5-cycloalkyl-2,2-dimethyl-pentan-3-onen und -olen mit pflanzenwachstumsregulierender und fungizider Wirksamkeit.

Überraschenderweise lassen sich pflanzenwachstumsregulierend und fungizid wirksame 4-Azolyl-5-cycloalkyl-2,2-dimethyl-pentan-3-one und -ole ausgehend von den erfindungsgemäßen substituierten 5-Cycloalkyl-2,2-dimethylpentan-3-onen der Formel (I) einfacher und in höherer Ausbeute herstellen als nach dem bisher bekannten Verfahren, bei dem die entsprechenden 4-Azolyl-2,2-dimethyl-butan-3-one als Zwischenprodukte eingesetzt wurden.

Die erfindungsgemäßen Stoffe sind durch die Formel (I) allgemein definiert.

Bevorzugt sind Verbindungen der Formel (I) in denen

R für gegebenenfalls durch Methyl substituiertes Cyclohexyl steht;

X für Fluor steht und

Y für Wasserstoff oder Fluor steht.

Verwendet man beispielsweise 2,2-Bisfluormethyl-5-phenyl-pent-4-en-3-on und Wasserstoff als Ausgangsstoffe sowie Palladium und Ruthenium auf Aktivkohle als Katalysatoren, so kann der Reaktionsablauf des erfindungsgemäßen Verfahrens durch das folgende Formelschema wiedergegeben werden:

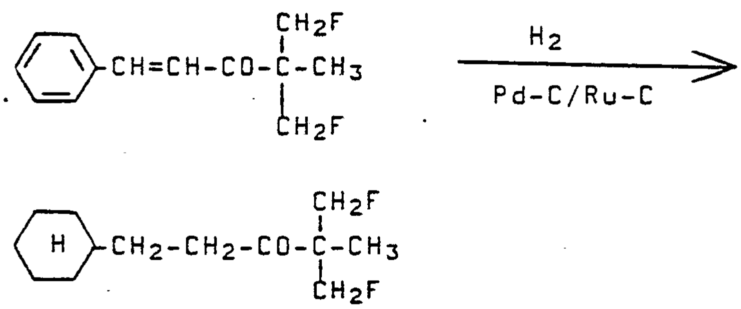


Verwendet man beispielsweise 2,2-Bisfluormethyl-5-(cyclohexen-1-yl)-pent-4-in-3-on und Wasserstoff als Ausgangsstoffe sowie Raney-Nickel als Katalysator, so kann der Reaktionsablauf des erfindungsgemäßen Verfahrens durch das folgende Formelschema wiedergegeben werden:

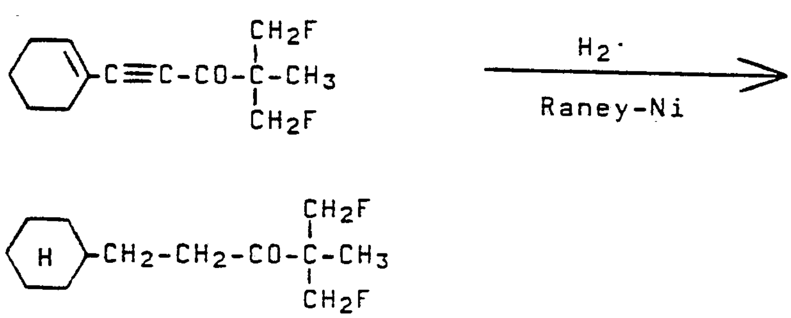


Verwendet man beispielsweise 2,2-Bisfluormethyl-5-phenyl-pentan-5-on und Wasserstoff als Ausgangsstoffe und Ruthenium auf Aktivkohle als Katalysator, so kann der Reaktionsablauf des erfindungsgemäßen Verfahrens durch das folgende Formelschema wiedergegeben werden:

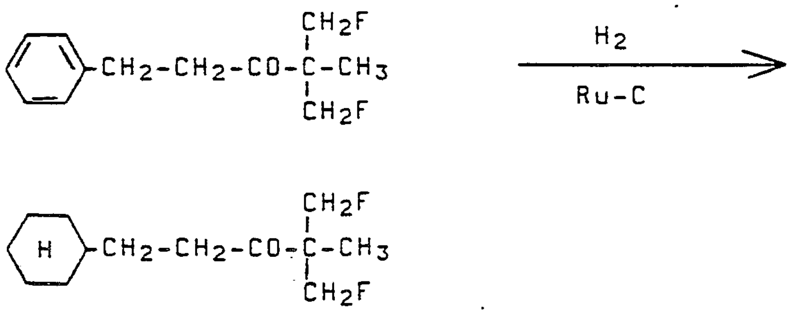


Die für die Durchführung des erfindungsgemäßen Verfahrens als Ausgangsstoffe zu verwendenden Ketone sind durch die Formeln (IIa), (IIb) und (IIc) allgemein definiert. In diesen Formeln steht $R^1$ für jeweils gegebenenfalls einfach bis dreifach, gleich oder verschieden durch Alkyl mit 1 bis 3 Kohlenstoffatomen substituiertes Cycloalkyl mit 5 bis 7 Kohlenstoffatomen, Cycloalkenyl mit 5 bis 7 Kohlenstoffatomen und Phenyl. $R^2$ steht für gegebenenfalls einfach bis dreifach, gleich oder verschieden durch Alkyl mit 1 bis 3 Kohlenstoffatomen substituiertes Phenyl.

Die Ketone der Formel (IIa), (IIb) und (IIc) sind noch nicht bekannt.

Die Ketone der Formel (IIa) lassen sich herstellen, indem man Butan-2-one der Formel (III)

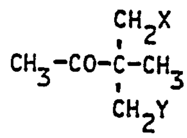

(III)

in welcher X und Y die oben angegebenen Bedeutungen haben, mit Aldehyden der Formel (IV)

$$R^1—CH=O \qquad (IV)$$

in welcher $R^1$ die oben angegebenen Bedeutungen hat, in Gegenwart eines Verdünnungsmittels, wie beispielsweise eines Alkoholes, und in Gegenwart einer Base, wie beispielsweise eines Alkalihydroxides oder -carbonates, bei Temperaturen zwischen 10 °C und 80 °C umsetzt.

Die Butan-2-one der Formel (III) und die Aldehyde der Formel (IV) sind bekannte Verbindungen der organischen Chemie.

Die Ketone der Formel (IIb) lassen sich herstellen, indem man Acetylen-Derivate der Formel (V)

$$R^1—C\equiv C—H \qquad (V)$$

in welcher $R^1$ die oben angegebenen bedeutungen hat, mit Pivalinsäurehalogeniden der Formel (VI),

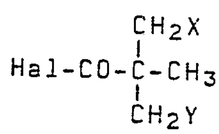

(VI)

in welcher

X und Y die oben angegebenen Bedeutungen haben und

Hal für Halogen, vorzugsweise Chlor oder Brom, steht,

in Gegenwart von Cu-(I)-Ionen als Katalysator und in Gegenwart eines Verdünnungsmittels, wie beispielsweise Toluol oder Pyridin, sowie in Gegenwart einer Base, wie beispielsweise Triethylamin, bei Temperaturen zwischen 20 °C und 100 °C umsetzt.

Die Acetylen-Derivate der Formel (V) und die Pivalinsäurehalogenide der Formel (VI) sind bekannte Verbindungen der organischen Chemie.

Die Ketone der Formel (IIc) lassen sich herstellen, indem man Ketone der Formeln

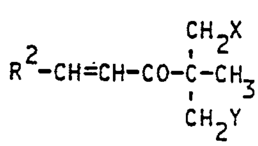

(IId)

oder

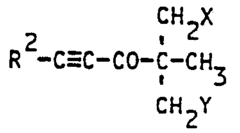

(IIe)

in welchen $R^2$, X und Y die oben angegebenen Bedeutungen haben,
mit Wasserstoff in Gegenwart eines Verdünnungsmittels, wie beispielsweise Methanol, und in Gegenwart eines Katalysators, wie beispielsweise Raney-Nickel oder Palladium auf Kohle unter Normaldruck oder unter erhöhtem Druck, wie vorzugsweise 30 bis 40 bar, bei Temperaturen zwischen 20 °C und 40 °C selektiv an der Doppelbindung bzw. Dreifachbindung hydriert.

Bei dem erfindungsgemäßen Verfahren arbeitet man in flüssiger Phase, vorzugsweise in Anwesenheit von Verdünnungsmitteln, unter Verwendung eines suspendierten, pulverförmigen Hydrierungskatalysators. Die Durchführung der erfindungsgemäßen Hydrierung kann diskontinuierlich (chargenweise) oder kontinuierlich als Sumpf- oder Rieselphasenhydrierung in bekannten Hydrierreaktoren, wie Autoklaven, Autoklavenkaskaden, Rohrreaktoren oder Umlaufreaktoren erfolgen. Die bevorzugte Arbeitsweise ist die diskontinuierliche Sumpfphasenhydrierung im Autoklaven bei erhöhtem Druck.

Als Verdünnungsmittel kommen bei der Durchführung des erfindungsgemäßen Verfahrens inerte organische Lösungsmittel in Frage. Hierzu gehören vorzugsweise Alkohole, wie Methanol, Ethanol, Isopropanol oder Ethylenglykol ; Ether, wie Diethylether, Diisopropylether, Ethylenglykolmonomethylether, Ethylenglykoldimethylether, Dioxan oder Tetrahydrofuran ; gesättigte Kohlenwasserstoffe, wie n-Heptan oder Cyclohexan ; sowie Ester, wie Essigsäureethylester.

Für das erfindungsgemäße Verfahren geeignete Hydrierkatalysatoren sind beispielsweise solche, die aus Metallen und/oder Verbindungen von Elementen der achten Nebengruppe des periodischen Systems der Elemente nach Mendelejew bestehen oder diese enthalten. Dabei sind die Metalle Ruthenium, Rhodium, Palladium, Platin, Kobalt und Nickel und deren Verbindungen bevorzugt. Bei den Metallverbindungen kann es sich beispielsweise um Oxide, Hydroxide und/oder Oxihydrate handeln. Zusätzlich können die Metalle Kupfer, Vanadium, Molybdän, Chrom und/oder Mangan, sowie Verbindungen dieser Metalle zugegen sein.

Die Hydrierkatalysatoren können ausschließlich oder überwiegend aus Wasserstoff-übertragenden Substanzen bestehen, diese können aber auch auf Trägermaterialien aufgebracht sein.

Als Trägermaterialien für die Wasserstoff-übertragenden Substanzen kommen beispielsweise in Frage : anorganische Materialien, wie Kieselgur, Kieselsäure, Aluminium-oxid, Alkali- und Erdalkalisilikate, Aluminiumsilikate, Montmorillonit, Zeolithe, Spinelle, Dolomit, Kaolin, Magnesiumsilikate, Zirkonoxid, Zinkoxid, Calciumcarbonat, Siliciumcarbid, Aluminiumphosphat, Borphosphat, Asbest, Aktivkohle oder Bariumsulfat, aber auch organische Materialien, beispielsweise natürlich vorkommende oder synthetische Verbindungen mit hohen Molekulargewichten wie Seide, Polyamide, Polystyrole, Zellstoff oder Polyurethane. Bevorzugt sind anorganische Trägermaterialien in Pulverform.

Derartige Trägerkatalysatoren können im allgemeinen 0,5 bis 50 Gew.-%, vorzugsweise 1 bis 10 Gew.-% der Wasserstoff-übertragenden Substanz, bezogen auf die Gesamtmasse des Trägerkatalysators, enthalten. Die Wasserstoff-übertragende Substanz kann dabei homogen im Trägermaterial verteilt sein, bevorzugt sind jedoch Katalysatoren, in deren äußerer Schicht oder auf deren Oberfläche die Wasserstoff-übertragende Substanz abgelagert ist. Die Herstellung und die Formgebung der Katalysatoren, die im erfindungsgemäßen Verfahren Verwendung finden können, kann in bekannter Weise erfolgen (siehe beispielsweise Houben-Weyl, Methoden der organischen Chemie, Band IV, Ic, Teil I, S. 16 bis 26, Georg Thieme Verlag, Stuttgart, 1980).

Bevorzugte Trägerkatalysatoren sind Ruthenium auf Kohle, Ruthenium auf Aluminiumoxid, Rhodium auf Kohle, Rhodium auf Aluminiumoxid, Palladium auf Kohle, Palladium auf Aluminiumoxid, Palladium auf Calciumcarbonat, Palladium auf Bariumsulfat, Palladium auf Kieselsäure, Platin auf Kohle und Platin auf Aluminiumoxid, Nickel auf Kieselgur, Nickel auf Aluminiumoxid sowie Nickel und Palladium auf Aluminiumoxid.

Bevorzugte Hydrierkatalysatoren, die ausschließlich oder überwiegend aus Wasserstoff-übertragender Substanz bestehen, sind beispielsweise oxidische Katalysatoren, wie Palladiumoxid, Platinoxid, Rutheniumoxid und/oder Rhodiumoxid/Platinoxid nach Nishimura, ferner durch Reduktion von entsprechenden Metallsalzen oder Metallsalzgemischen mit Alkalihydriden, Alkaliboranaten, Metallalkylen, Hydrazin, Formaldehyd, Wasserstoff oder elektropositiven Metallen herstellbare Schwarzkatalysatoren, wie Palladium/Schwarz, Platin/Schwarz und Rhodium/Schwarz ; sowie Skelettkatalysatoren vom Raney-Typ, wie Raney-Nickel, Raney-Kobalt, Raney-Nickel-Kobalt, Raney-Nickel-Eisen, Raney-Nickel-Kupfer, Raney-Nickel-Eisen-Chrom, Raney-Nickel-Palladium und Raney-Nickel-Eisen-Vanadium.

Die Auswahl eines oder mehrerer der genannten Hydrierkatalysatoren richtet sich zweckmäßigerweise nach der Konstitution der erfindungsgemäß zu hydrierenden Ausgangsketone der Formeln (IIa), (IIb) und (IIc).

Enthalten die Ketone der Formeln (IIa) und (IIb) als Substituent $R^1$ gegebenenfalls substituierte Cycloalkenylreste oder gegebenenfalls substituierte Cycloalkylreste, so sind zu deren Überführung in gesättigte Ketone der Formel (I) diejenigen Katalysatoren, die Nickel und/oder Palladium enthalten oder daraus bestehen, besonders bevorzugt.

Enthalten die Ketone der Formeln (IIa) und (IIb) als Substituenten $R^1$ gegebenenfalls substituierte Arylreste oder handelt es sich um ein Keton der Formel (IIc), so sind zu deren Überführung in gesättigte Ketone der Formel (I) diejenigen Katalysatoren, die Ruthenium, Rhodium und/oder Platin enthalten oder daraus bestehen, besonders bevorzugt.

Die Hydrierkatalysatoren werden im erfindungsgemäßen Verfahren in einer solchen Menge eingesetzt, daß 0,05 bis 2,5, vorzugsweise 0,1 bis 1 Gew.-% Wasserstoff-übertragende Substanz bezogen auf das Gesamtgewicht des Reaktionsgemisches vorliegen.

Zur Durchführung des erfindungsgemäßen Verfahrens können auch Gemische aus zwei oder mehreren der genannten Hydrierkatalysatoren verwendet werden.

Die katalytische Aktivität der Hydrierkatalysatoren bleibt bei der Durchführung des erfindungsgemäßen Verfahrens im allgemeinen weitgehend erhalten, so daß diese bei diskontinuierlicher Arbeitsweise wiederholt eingesetzt werden können und bei kontinuierlicher Arbeitsweise längere Zeit in Gebrauch bleiben können.

Die Reaktionstemperaturen können in einem größeren Bereich variiert werden. Im allgemeinen arbeitet man im Bereich zwischen 0 °C und 150 °C, vorzugsweise zwischen 20 °C und 120 °C. Für die Hydrierung von aliphatischen und/oder cycloaliphatischen C-C-Mehrfachbindungen in den Ketonen der Formeln (IIa) und (IIb) mit den dafür bevorzugten Katalysatoren sind Reaktionstemperaturen im Bereich von 20 °C bis 60 °C besonders bevorzugt, während für die Hydrierung von Arylresten in den Ketonen der Formeln (IIa), (IIb) und (IIc) mit den dafür bevorzugten Katalysatoren Temperaturen im Bereich von 60 °C bis 120 °C besonders bevorzugt sind.

Die erfindungsgemäßen Hydrierungen werden vorzugsweise bei erhöhtem Druck durchgeführt. Im allgemeinen arbeitet man zwischen 1 und 150 bar, vorzugsweise bei 20 bis 120 bar. Für die Hydrierung von aliphatischen und/oder cycloaliphatischen C-C-Mehrfachbindungen in den Ketonen der Formeln (IIa) und (IIb) mit den dafür bevorzugten Katalysatoren sind Drucke im Bereich von 5 bis 50 bar besonders bevorzugt, während für die Hydrierung von Arylresten in den Ketonen der Formeln (IIa), (IIb) und (IIc) mit den dafür bevorzugten Katalysatoren Drucke im Bereich von 5 bis 120 bar besonders bevorzugt sind.

Die für das erfindungsgemäße Verfahren erforderliche Reaktionszeit ist abhängig von der Reaktionstemperatur, dem Wasserstoffpartialdruck, der Intensität der Durchmischung des Reaktionsgemisches und von der Aktivität und Konzentration des Hydrierkatalysators. Im allgemeinen liegt die erforderliche Reaktionszeit im Bereich von 15 Minuten bis zu mehreren Stunden.

Das erfindungsgemäße Verfahren kann beispielsweise in der einfachsten Ausführungsform diskontinuierlich in folgender Weise durchgeführt werden: Ein mit einer Rühr- oder Mischeinrichtung versehener, temperierbarer Autoklav wird in geeigneter Weise mit einem Keton der Formel (IIa), (IIb) oder (IIc), dem Hydrierungskatalysator und dem Verdünnungsmittel beschickt. Nachdem der Autoklav entlüftet und sodann Wasserstoff bis zu dem gewünschten Druck aufgedrückt worden ist, wird das Gemisch unter intensiver Durchmischung auf die gewählte Reaktionstemperatur erhitzt. Der Reaktionsverlauf läßt sich leicht durch Messung des Wasserstoffverbrauches, der durch weitere Wasserstoffzufuhr ausgeglichen wird, verfolgen. Die Hydrierung ist beendet, wenn kein Wasserstoff mehr verbraucht wird und die verbrauchte Wasserstoffmenge etwa der theoretisch erforderlichen Wasserstoffmenge entspricht.

Nach beendeter Hydrierung wird das Reaktionsgemisch abgekühlt, entspannt und in bekannter Weise, beispielsweise durch Abfiltrieren des Katalysators und Destillieren des Verdünnungsmittels, aufgearbeitet.

In einer besonderen Ausführungsform der erfindungsgemäßen Umsetzung wird so verfahren, daß in einer ersten Stufe Ketone der Formel (IIa), die als Substituenten $R^1$ einen gegebenenfalls substituierten Arylrest tragen, zu entsprechenden Ketonen der Formel (IIc) hydriert werden, die dann in einer zweiten Stufe zu den Endprodukten der Formel (I) hydriert werden (vgl. auch die Herstellungsbeispiele). Hervorzuheben ist, daß mit hoher Selektivität nur die C-C-Mehrfachbindungen hydriert werden, während die CO-Doppelbindung erhalten bleibt.

Wie schon erwähnt, stellen die neuen 5-Cycloalkyl-2,2-dimethyl-pentan-3-one der Formel (I) interessante Zwischenprodukte zur Synthese von 4-Azolyl-5-cycloalkyl-2,2-dimethyl-pentan-3-onen und -olen mit fungiziden und pflanzenwuchsregulierenden Eigenschaften dar.

Solche 4-Azolyl-5-cycloalkyl-2,2-dimethyl-pentan-3-one und -ole der Formel (VII)

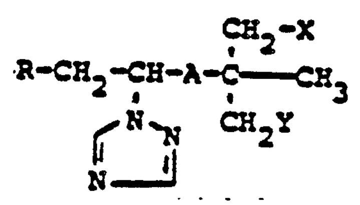

(VII)

in welcher

R, X und Y die oben angegebenen Bedeutungen haben und

A für eine Keto- oder die CH(OH)-Gruppe steht,

lassen sich herstellen, indem man 5-Cycloalkyl-2,2-dimethyl-pentan-3-one der Formel (I)

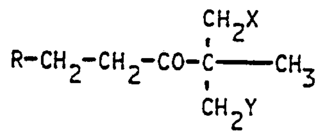

(I)

in welcher R, X und Y die oben angegebenen Bedeutungen haben, mit Chlor oder Brom in Gegenwart eines inerten organischen Lösungsmittels, wie beispielsweise Ether, chlorierten oder nicht chlorierten Kohlenwasserstoffen, bei Raumtemperatur umsetzt, oder mit üblichen Chlorierungsmitteln, wie beispielsweise Sulfurylchlorid, bei 20 °C bis 60 °C umsetzt ; anschließend die so erhaltenen Halogenketone der Formel (VIII)

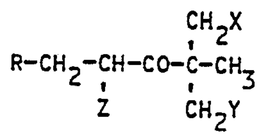

(VIII)

in welcher

R, X und Y die oben angegebenen Bedeutungen haben und

Z für Chlor oder Brom steht,

mit 1,2,4-Triazol in Gegenwart eines inerten organischen Lösungsmittels, wie beispielsweise Acetonitril, und in Gegenwart eines Säurebindemittels, wie beispielsweise Kaliumcarbonat oder in Gegenwart eines Überschusses an 1,2,4-Triazol, bei Temperaturen zwischen 60 °C und 120 °C umsetzt ;

und gegebenenfalls anschließend die so erhaltenen 4-Azolyl-5-cycloalkyl-2,2-dimethyl-pentan-3-one der Formel (VIIa)

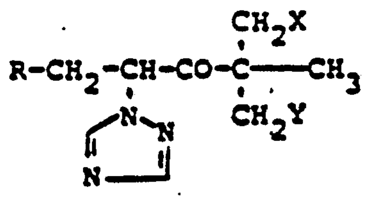

(VIIa)

in welcher R, X und Y die oben angegebenen Bedeutungen haben, durch Umsetzung mit komplexen Hydriden, wie Natriumborhydrid oder Lithiumalanat, in Gegenwart eines polaren organischen Lösungsmittels, wie z. B. eines Alkoholes, bei Temperaturen zwischen 0 °C und 30 °C reduziert ; oder durch Umsetzung mit Aluminiumisopropylat in Gegenwart eines Verdünnungsmittels, wie z. B. Isopropanol, bei Temperaturen von 20 °C bis 120 °C reduziert.

Die 4-Azolyl-5-cycloalkyl-2,2-dimethyl-pentan-3-one und -ole der Formel (VII) weisen starke fungizide und pflanzenwuchsregulierende Eigenschaften auf (vgl. EP-B 0 031 911 und EP-B 0 032 200).

Die Herstellung und die Verwendung der erfindungsgemäßen Substanzen geht aus den folgenden Beispielen hervor.

## Herstellungsbeispiele

### Beispiel 1

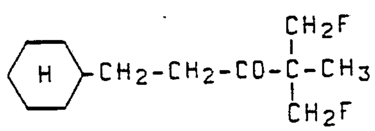

(I-1)

Verfahrensvariante 1

1. Stufe :

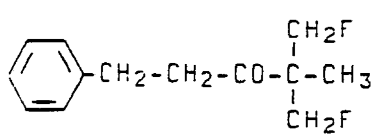

(II-1)

Ein 120 l Rührautoklav aus Edelstahl, der mit Hilfe eines regelbaren Thermostaten temperiert werden kann, wurde mit 30 kg (133,9 Mol) 2,2-Bisfluormethyl-5-phenyl-pent-4-en-3-on, 60 l Methanol und 0,6 kg Raney-Nickel beschickt.

Nach Verschließen des Autoklaven und Verdrängen der Luft mit Stickstoff wurde das eingesetzte Gemisch mit Wasserstoff bis zu einem Druck von 30 bar beaufschlagt und unter Rühren auf 30 °C erhitzt. Sobald diese Temperatur erreicht war, wurde des Wasserstoffdruck auf 40 bar erhöht und nach Maßgabe des Wasserstoff-Verbrauches während der gesamten Reaktionszeit aufrechterhalten.

Nachdem die Wasserstoffaufnahme nach etwa 5 Stunden beendet war, wurde zur Vervollständigung der Umsetzung noch eine Stunde unter den vorangenannten Hydrierbedingungen weiter gerührt, anschließend auf Raumtemperatur abgekühlt und auf Normaldruck entspannt.

Die durch Filtration vom Katalysator abgetrennte Produktlösung wurde ohne Isolierung direkt weiter in der 2. Stufe umgesetzt. Das erhaltene 2,2-Bisfluormethyl-5-phenyl-pentan-3-on hat einen Gehalt von 98 % (gaschromatographisch bestimmt).

2. Stufe :

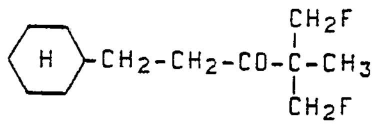

(I-1)

Ein 120 l Rührautoklav aus Edelstahl, der mit Hilfe eines regelbaren Thermostaten temperiert werden kann, wurde mit einer Lösung von 29,7 kg (131,4 Mol) 2,2-Bisfluormethyl-5-phenyl-pentan-3-on in 60 l Methanol und 0,72 kg eines 5 % Ruthenium auf Aktivkohle enthaltenden Katalysators beschickt.

Nach Verschließen des Autoklaven und Verdrängen der Luft mit Stickstoff wurde das eingesetzte Gemisch mit Wasserstoff bis zu einem Druck von 50 bar beaufschlagt und unter Rühren auf 90 °C erhitzt. Sobald diese Temperatur erreicht war, wurde der Wasserstoffdruck auf 100 bar erhöht und nach Maßgabe des Wasserstoff-Verbrauches während der gesamten Reaktionszeit aufrechterhalten.

Nachdem die Wasserstoffaufnahme nach etwa 6 Stunden beendet war, wurde zur Vervollständigung der Umsetzung noch eine Stunde unter den vorangenannten Hydrierbedingungen weiter gerührt, anschließend auf Raumtemperatur abgekühlt und auf Normaldruck entspannt.

Die durch Filtration vom Katalysator abgetrennte Produktlösung wurde im Rotationsverdampfer vom Methanol befreit.

Man erhielt 30,3 kg (99,4 % der Theorie) 2,2-Bisfluormethyl-5-cyclohexyl-pentan-3-on als Öl mit einem Gehalt von 96 % (gaschromatographisch bestimmt).

Herstellung des Ausgangsproduktes :

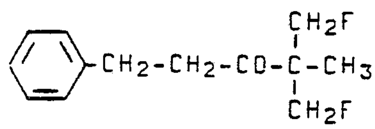

(II-1)

Eine Mischung von 106,1 g (1 Mol) Benzaldehyd, 135,1 g (1 Mol) 3,3-Bisfluormethyl-butan-2-on, 300 g Methanol und 40 g (1 Mol) Natriumhydroxid in 70 g Wasser wurde 3 Stunden bei Raumtemperatur gerührt. Anschließend wurde das kristalline Produkt abfiltriert und getrocknet.

Man erhielt 201,8 g (90 % der Theorie) 2,2-Bisfluormethyl-5-phenyl-pent-4-en-3-on vom Schmelzpunkt 45 °C.

Beispiel 2

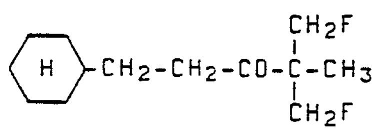

(I-1)

Verfahrensvariante 2

Ein 0,7 l Rührautoklav aus Edelstahl, der mit Hilfe eines regelbaren Thermostaten temperiert werden kann, wurde mit 112 g (0,5 Mol) 2,2-Bisfluormethyl-5-phenyl-pent-4-en-3-on, 300 ml Methanol, 2,8 g eines 5 % Palladium auf Aktivkohle enthaltenden Katalysators und 2,8 g eines 5 % Ruthenium auf Aktivkohle enthaltenden Katalysators beschickt.

Nach Verschließen des Autoklaven und Verdrängen der Luft durch Stickstoff wurde Wasserstoff bis zu einem Druck von 30 bar zugeführt und unter Rühren auf 40 °C aufgeheizt. Sobald diese Temperatur erreicht war, wurde der Wasserstoffdruck auf 50 bar erhöht und in dieser Höhe bis zum Abklingen der Wasserstoffaufnahme aufrechterhalten ($H_2$-Verbrauch etwa 0,5 Mol in 1,5 Stunden). Anschließend wurde die Reaktionslösung auf 70 °C und der Wasserstoffdruck auf 100 bar erhöht und die Hydrierung unter diesen Bedingungen fortgesetzt, indem ständig Wasserstoff nach Maßgabe des am Druckabfall erkennbaren Wasserstoff-Verbrauchs bis zu einem Druck von 100 bar nachgedrückt wurde ($H_2$-Verbrauch etwa 1,5 Mol in 5 Stunden).

Nachdem die Wasserstoffaufnahme beendet war, wurde auf Raumtemperatur abgekühlt und auf Normaldruck entspannt. Die durch Filtration vom Katalysator abgetrennte Produktlösung wurde im Rotationsverdampfer von Methanol befreit.

Man erhielt 113 g (97,4 % der Theorie) 2,2-Bisfluormethyl-5-cyclohexyl-pentan-3-on als Öl mit einem Gehalt von 89 % (gaschromatographisch bestimmt).

Beispiel 3

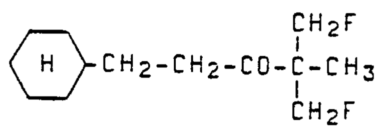

(I-1)

Verfahrensvariante 3

1. Stufe :

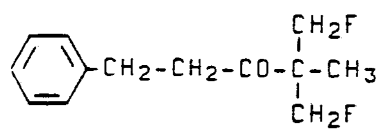

(II-1)

Ein 0,3 l Rührautoklav aus Edelstahl, der mit Hilfe eines regelbaren Thermostaten temperiert werden kann, wurde mit 44,4 g (0,2 Mol) 2,2-Bisfluormethyl-5-phenyl-pent-4-in-3-on, 170 ml Methanol und 5 g Raney-Nickel beschickt.

Nach Verschließen des Autoklaven und Verdrängen der Luft mit Stickstoff wurde das eingesetzte Gemisch mit Wasserstoff bis zu einem Druck von 30 bar beaufschlagt und unter Rühren auf 30 °C erhitzt. Sobald diese Temperatur erreicht war, wurde der Wasserstoffdruck auf 50 bar erhöht und nach Maßgabe des Wasserstoff-Verbrauches während der gesamten Reaktionszeit aufrechterhalten.

Nachdem die Wasserstoffaufnahme nach etwa 2 Stunden beendet war, wurde zur Vervollständigung der Umsetzung noch eine Stunde unter den vorangenannten Hydrierbedingungen weiter gerührt, anschließend auf Raumtemperatur abgekühlt und auf Normaldruck entspannt.

Die durch Filtration vom Katalysator abgetrennte Produktlösung wurde im Rotationsverdampfer vom Methanol befreit.

Man erhielt 44,5 g (98,5 % der Theorie) 2,2-Bisfluormethyl-5-phenyl-pentan-3-on als Öl einem Gehalt von 96,5 % (gaschromatographisch bestimmt).

2. Stufe :

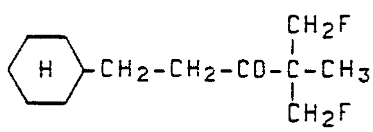

(I-1)

Die Umsetzung verlaüft entsprechend der 2. Stufe bei Verfahrensvariante 1.

Herstellung des Ausgangsproduktes

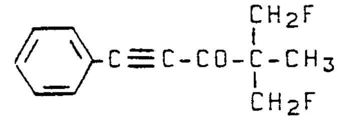

(II-3)

In 30 ml Pyridin legte man unter Stickstoff 10,1 g (0,1 Mol) Triethylamin und 1,43 g (0,01 Mol) Kupfer-(I)-bromid vor. Man fügte 10,2 g (0,1 Mol) Phenylacetylen zu und ließ 30 Minuten nachrühren. Danach versetzte man die Reaktionsmischung tropfenweise mit 15,6 g (0,1 Mol) α,α-Bisfluormethyl-propionsäurechlorid und hielt die Temperatur dabei auf 60 °C. Man ließ 15 Stunden bei dieser Temperatur rühren, kühlte ab, wusch mit Wasser, trocknete über Natriumsulfat und engte im Vakuum ein. Der Rückstand wurde durch Destillation gereinigt.

Man erhielt 17,3 g (78 % der Theorie) 2,2-Bisfluormethyl-5-phenyl-pent-4-in-3-on vom Siedepunkt 103 °C bis 105 °C/0,2 mbar.

Beispiel 4

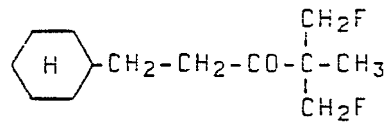

(I-1)

Verfahrensvariante 4

Ein 0,3 l Rührautoklav aus Edelstahl, der mit Hilfe eines regelbaren Thermostaten temperiert werden kann, wurde mit 24 g (0,106 Mol) 2,2-Bisfluormethyl-5-(cyclohexen-1-yl)-pent-4-in-3-on, 120 ml Methanol und 5 g Raney-Nickel beschickt.

Nach Verschließen des Autoklaven und Verdrängen der Luft mit Stickstoff wurde das eingesetzte Gemisch mit Wasserstoff bis zu einem Druck von 50 bar beaufschlagt und unter Rühren auf 50 °C erhitzt. Sobald diese Temperatur erreicht war, wurde der Wasserstoffdruck auf 70 bar erhöht und nach Maßgabe des Wasserstoff-Verbrauches während der gesamten Reaktionszeit aufrechterhalten.

Nachdem die Wasserstoffaufnahme beendet war, wurde zur Vervollständigung der Umsetzung noch eine Stunde unter den vorangenannten Hydrierbedingungen weiter gerührt, anschließend auf Raumtemperatur abgekühlt und auf Normaldruck entspannt.

Die durch Filtration vom Katalysator abgetrennte Produktlösung wurde am Rotationsverdampfer vom Methanol befreit.

Man erhielt 23,5 g (95,5% der Theorie) 2,2-Bisfluormethyl-5-cyclohexyl-pentan-3-on als Öl mit einem Gehalt von 88,5 % (gaschromatographisch bestimmt).

Herstellung des Ausgangsproduktes

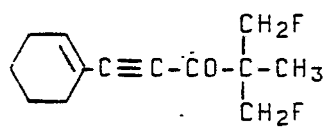

(II-4)

In 30 ml Pyridin legte man unter Stickstoff 10,1 g (0,1 Mol) Triethylamin und 1,43 g (0,01 Mol) Kupfer-(I)-bromid vor. Man fügte 10,6 g (0,1 Mol) Cyclohexen-1-yl-acetylen zu und ließ 30 Minuten nachrühren. Danach versetzte man die Reaktionsmischung tropfenweise mit 15,6 g (0,1 Mol) α,α-Bis-fluormethyl-propionsäurechlorid und hielt die Temperatur auf 70 °C. Man ließ 15 Stunden bei dieser Temperatur nachrühren, kühlte ab, wusch mit Wasser, trocknete über Natriumsulfat und engte im Vakuum ein. Der Rückstand wurde durch Destillation gereinigt.

Man erhielt 18,1 g (80 % der Theorie) 2,2-Bisfluormethyl-5-(cyclohexen-1-yl)-pent-4-in-3-on vom Siedepunkt 106 °C bis 109 °C/0,3 mbar.

Herstellung von 4-Azolyl-5-cycloalkyl-2,2-dimethyl-pentan-3-onen der Formel (VII)

Beispiel 5

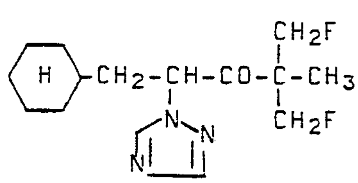

(VII-1)

1. Stufe

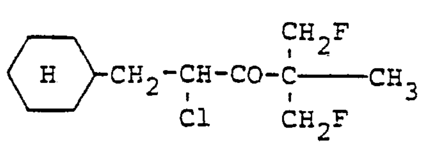

(VIII-1)

232,3 g (1 Mol) 2,2-Bisfluormethyl-5-cyclohexyl-pentan-3-on (Beispiel 1) wurden auf 80 °C erhitzt und innerhalb eine Stunde tropfenweise mit 161,9 g (1,2 Mol) Sulfurylchlorid versetzt. Man ließ 5 Stunden bei 80 °C nachrühren und destillierte anschließend überschüssiges Sulfurylchlorid im Vakuum ab. Nach dem Abkühlen auf 20 °C wurde mit 500 ml Methylisobutylketon versetzt. Die organische Lösung wurde mit Wasser neutral gewaschen, über Magnesiumsulfat getrocknet und im Vakuum eingeengt. Der Rückstand wurde destilliert.

Man erhielt 252 g (90 % der Theorie) 2,2-Bisfluormethyl-5-chlor-5-cyclohexyl-pentan-3-on vom Siedepunkt 118 °C bis 120 °C/2,5 mbar.

2. Stufe

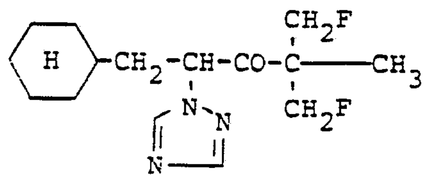

(VII-1)

266,7 g (1 Mol) 2,2-Bisfluormethyl-4-chlor-5-cyclohexyl-pentan-3-on, 69,1 g (1 Mol) 1,2,4-Triazol und 165,8 g (1,2 Mol) Kaliumcarbonat in 1 000 ml Methylisobutylketon wurden 6 Stunden unter Rückfluß erhitzt. Nach dem Abkühlen wurde mit verdünnter Salzsäure und mit Wasser neutral gewaschen. Die organische Phase wurde über Magnesiumsulfat getrocknet und im Vakuum eingeengt.

Man erhielt 329 g (88 % der Theorie) 2,2-Bisfluormethyl-5-cyclohexyl-4-(1,2,4-triazol-1-yl)-pentan-3-on vom Brechungsindex $n_D^{20}$ = 1,493 3.

Beispiel 6

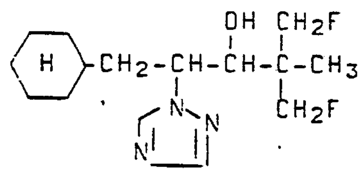

(VII-2)

299 g (1 Mol) 2,2-Bisfluormethyl-5-cyclohexyl-4-(1,2,4-triazol-1-yl)-pentan-3-on (Beispiel 5) wurden in 300 ml Methanol gelöst und bei 0 °C bis 5 °C tropfenweise mit einer Lösung von 13,2 g (0,35 Mol) Natriumborhydrid in 150 ml 0,1 normaler wäßriger Natronlauge versetzt. Nach einer Reaktionszeit von 2 Stunden wurde die Reaktionslösung mit verdünnter wäßriger Salzsäure auf einen pH-Wert von 4 bis 5 eingestellt. Nach Zugabe von 500 ml Wasser kristallisierte das Endprodukt aus.

Nach Trocknung im Vakuum erhielt man 286 g (95 % der Theorie) 2,2-Bisfluormethyl-5-cyclohexyl-4-(1,2,4-triazol-1-yl)-pentan-3-ol vom Schmelzpunkt 103 °C bis 105 °C.

Vergleichsbeispiel

Herstellung des (1,2,4-Triazol-1-yl)-Derivates der Formel

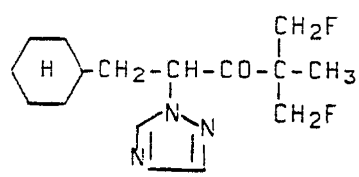

(VII-1)

nach dem bisher bekannten Verfahren.

1. Stufe

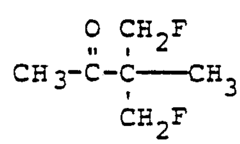

In einem Dreihalskolben mit Rührer, Tropftrichter und Liebigkühler mit gekühlter Vorlage wurden 400 ml Tetraethylenglykol und 46,4 g Kaliumfluorid (0,8 Mol) vorgelegt und auf 170 °C aufgeheizt. Man

legte an den Vorstoß des Liebigkühlers ein Wasserstrahlvakuum (Druck ca. 20 bis 30 mbar) an. Dann wurden innerhalb von 45 Minuten 57,6 g (0,2 Mol) 2-Acetyl-2-methyl-propan-1,3-diol-bismethansulfat, gelöst in 100 ml Tetraethylenglykol, zugetropft. Das entstandene 3,3-Bisfluormethyl-butan-2-on wurde während der Reaktion in die gekühlte Vorlage abdestilliert. Nach dem Zutropfen wurde noch 1 Stunden bei 175 °C weiter destilliert. Das aufgefangene Destillat wurde anschließend redestilliert. Man erhielt 14 g (51,5 % der Theorie) 3,3-Bisfluormethylbutan-2-on vom Kp. 43 bis 46 °C/12 mbar.

2. Stufe

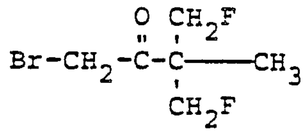

136 g (1 Mol) 3,3-Bisfluormethylbutan-2-on in 700 ml Methylenchlorid wurden tropfenweise so mit 51 ml (1 Mol) Brom versetzt, daß laufend Entfärbung eintrat. Anschließend wurde das Lösungsmittel im Wasserstrahlvakuum abdestilliert. Man erhielt nahezu quantitativ rohes 3,3-Bisfluormethyl-1-brom-butan-2-on als Öl, das direkt weiter umgesetzt werden konnte.

3. Stufe

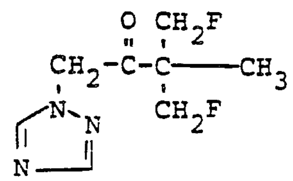

215 g (1 Mol) rohes 3,3-Bisfluormethyl-1-brom-butan-2-on wurden bei 30 bis 35 °C zu 84 g (1,2 Mol) 1,2,4-Triazol und 165 g (1,2 Mol) gemahlenem Kaliumcarbonat in 1 l Ethanol getropft. Man ließ über Nacht bei 40 °C nachrühren, filtrierte danach vom Ungelösten ab und engte das Filtrat ein. Der ölige Rückstand wurde mit Methylenchlorid und Wasser extrahiert, über Natriumsulfat getrocknet und eingeengt. Der Rückstand wurde in Methylenchlorid aufgenommen und mit 140 ml etherischer Salzsäure versetzt. Das entstandene kristalline Produkt wurde abgesaugt, mit 1 l Methylenchlorid und 1 l gesättigter, wäßriger Natriumhydrogencarbonatlösung ausgerührt, mit 1 Liter Wasser gewaschen, über Natriumsulfat getrocknet und eingeengt. Man erhielt 73,8 g (36,4 % der Theorie) 3,3-Bisfluormethyl-1-(1,2,4-triazol-1-yl)-butan-2-on als Öl, das direkt weiter umgesetzt werden konnte.

4. Stufe

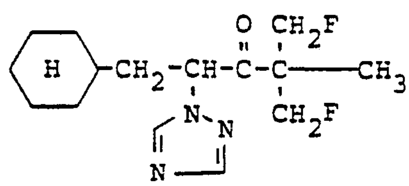

Eine Lösung von 101,4 g (1,81 Mol) Kaliumhydroxid in 217,2 ml Wasser wurde bei Raumtemperatur unter Rühren in eine Lösung von 369,4 g (1,81 Mol) 2,2-Bis-fluormethyl-4-(1,2,4-triazol-1-yl)-butan-3-on in 2 Litern Dimethylsulfoxid gegeben. In dieses Gemisch wurde 320,5 g (1,81 Mol) Cyclohexylmethylbromid unter Rühren tropfenweise zugegegeben, wobei die Temperatur des Reaktionsgemisches durch Kühlung zwischen 20 und 40 °C gehalten wurde. Das Reaktionsgemisch wurde noch 15 Stunden bei 60 °C gerührt und dann in 2 Liter Wasser gegossen. Man extrahierte das erhaltene Gemisch zweimal mit je 1 Liter Methylenchlorid, wusch die vereinigten organischen Phasen viermal mit je 2 Litern Wasser, trocknete über Natriumsulfat und zog das Lösungsmittel ab. Das vorliegende ölige Produkt wurde in Aceton aufgenommen, und 326 g Naphthalin-1,5-disulfonsäure wurden zu der Lösung hinzugefügt. Der sich dabei bildende Niederschlag wurde abgesaugt und in 2 Litern Methylenchlorid suspendiert. Diese Suspension wurde zweimal mit je 2 Litern gesättigter, wäßriger Natriumhydrogencarbonat-Lösung geschüttelt. Dann wurde die organische Phase mit 2 Litern Wasser gewaschen und nach dem Trocknen über Natriumsulfat unter vermindertem Druck eingeengt. Man erhielt auf diese Weise 297,5 g (63 % der Theorie) an 2,2-Bis-fluormethyl-5-cyclohexyl-4-(1,2,4-triazol-1-yl)-pentan-3-on in Form eines Öles. $n_D^{20}$ = 1,483 7.

**Patentansprüche**

1. Substituierte 5-Cycloalkyl-2,2-dimethyl-pentan-3-one der Formel

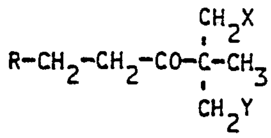

(I)

in welcher

R für gegebenenfalls einfach bis dreifach, gleich oder verschieden durch Alkyl mit 1 bis 3 Kohlenstoffatomen substituiertes Cycloalkyl mit 5 bis 7 Kohlenstoffatomen steht,

X für Fluor oder Chlor steht und

Y für Wasserstoff, Fluor oder Chlor steht.

2. Verfahren zur Herstellung von substituierten 5-Cycloalkyl-2,2-dimethyl-pentan-3-onen der Formel

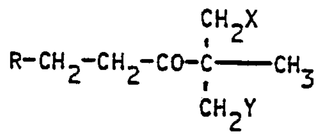

(I)

in welcher

R für gegebenenfalls einfach bis dreifach, gleich oder verschieden durch Alkyl mit 1 bis 3 Kohlenstoffatomen substituiertes Cycloalkyl mit 5 bis 7 Kohlenstoffatomen steht,

X für Fluor oder Chlor steht und

Y für Wasserstoff, Fluor oder Chlor steht,

dadurch gekennzeichnet, daß man Ketone der Formeln

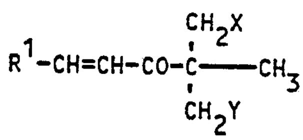

(IIa)

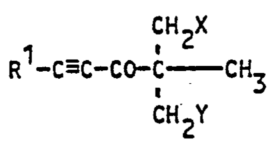

(IIb)

oder

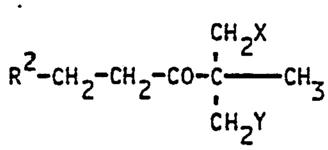

(IIc)

in welchen

$R^1$ für jeweils gegebenenfalls einfach bis dreifach, gleich oder verschieden durch Alkyl mit 1 bis 3 Kohlenstoffatomen substituiertes Cycloalkyl mit 5 bis 7 Kohlenstoffatomen, Cycloalkenyl mit 5 bis 7 Kohlenstofatomen oder Phenyl steht,

$R^2$ für gegebenenfalls einfach bis dreifach, gleich oder verschieden durch Alkyl mit 1 bis 3 Kohlenstoffatomen substituiertes Phenyl steht und

X und Y die oben angegebenen Bedeutungen haben,

selektiv mit Wasserstoff in Gegenwart eines Hydrierkatalysators sowie gegebenenfalls in Gegenwart eines Verdünnungsmittels umsetzt.

3. Verfahren zur Herstellung von 4-Azolyl-5-cycloalkyl-2,2-dimethyl-pentan-3-onen und -olen der Formel

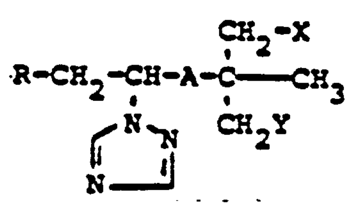

(VII)

in welcher

R für gegebenenfalls einfach bis dreifach, gleich oder verschieden durch Alkyl mit 1 bis 3 Kohlenstoffatomen substituiertes Cycloalkyl mit 5 bis 7 Kohlenstoffatomen steht,

X für Fluor oder Chlor steht und

Y für Wasserstoff, Fluor oder Chlor steht,

A für eine Keto- oder eine —CH(OH)-Gruppe steht,

dadurch gekennzeichnet, daß man sbstituierte 5-Cycloalkyl-2,2-dimethyl-pentan-3-one der Formel

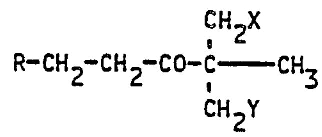

(I)

in welcher R, X und Y die oben angegebenen Bedeutungen haben,

mit Chlor oder Brom in Gegenwart eines inerten organischen Lösungsmittels, oder mit Chlorierungsmitteln gegebenenfalls in Gegenwart eines Verdünnungsmittels umsetzt, danach die so erhaltenen Halogenketone der Formel

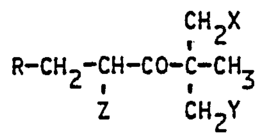

(VIII)

in welcher

R, X und Y die oben angegebene Bedeutungen haben und

Z für Chlor oder Brom steht,

mit 1,2,4-Triazol in Gegenwart eines inerten organischen Lösungsmittels umsetzt, und gegebenenfalls anschließend die so erhaltenen 4-Azolyl-5-cycloalkyl-2,2-dimethylpentan-3-one der Formel

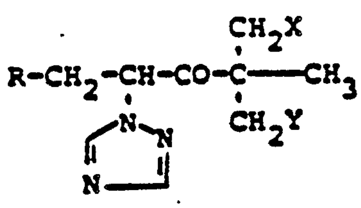

(VIIa)

in welcher R, X und Y die oben angegebenen Bedeutungen haben,

entweder mit komplexen Hydriden in Gegenwart eines polaren organischen Lösungsmittels, oder mit Aluminiumisopropylat in Gegenwart eines Verdünnungsmittels umsetzt.

4. Ketone der Formel

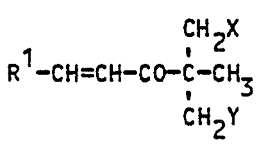

(IIa)

in welcher

$R^1$ für jeweils gegebenenfalls einfach bis dreifach, gleich oder verschieden durch Alkyl mit 1 bis 3 Kohlenstoffatomen substituiertes Cycloalkyl mit 5 bis 7 Kohlenstoffatomen, Cycloalkenyl mit 5 bis 7 Kohlenstoffatomen oder Phenyl steht,

X für Fluor oder chlor steht und

Y für Wasserstoff, Fluor oder Chlor steht.

5. Verfahren zur Herstellung von Ketonen der Formel

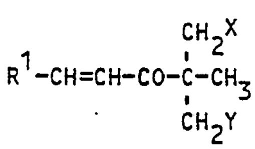

(IIa)

in welcher

$R^1$ für jeweils gegebenenfalls einfach bis dreifach, gleich oder verschieden durch Alkyl mit 1 bis 3 Kohlenstoffatomen substituiertes Cycloalkyl mit 5 bis 7 Kohlenstoffatomen, Cycloalkenyl mit 5 bis 7 Kohlenstoffatomen oder Phenyl steht,

X für Fluor oder Chlor steht und

Y für Wasserstoff, Fluor oder Chlor steht,

dadurch gekennzeichnet, daß man Butan-2-one der Formel

$$CH_3-CO-\underset{CH_2Y}{\overset{CH_2X}{C}}-CH_3 \qquad \text{(III)}$$

in welcher X und Y die oben angegebenen Bedeutungen haben, mit Aldehyden der Formel

$$R^1-CH=O \qquad \text{(IV)}$$

in welcher $R^1$ die oben angegebenen Bedeutungen hat, in Gegenwart eines Verdünnungsmittels und in Gegenwart einer Base umsetzt.

6. Ketone der Formel

$$R^2-CH_2-CH_2-CO-\underset{CH_2Y}{\overset{CH_2X}{C}}-CH_3 \qquad \text{(IIc)}$$

in welcher

$R^2$ für gegebenenfalls einfach bis dreifach, gleich oder verschieden durch Alkyl mit 1 bis 3 Kohlenstoffatomen substituiertes Phenyl steht,

X für Fluor oder Chlor steht und

Y für Wasserstoff, Fluor oder Chlor steht.

7. Verfahren zur Herstellung von Ketonen der Formel

$$R^2-CH_2-CH_2-CO-\underset{CH_2Y}{\overset{CH_2X}{C}}-CH_3 \qquad \text{(IIc)}$$

in welcher

$R^2$ für gegebenenfalls einfach bis dreifach, gleich oder verschieden durch Alkyl mit 1 bis 3 Kohlenstoffatomen substituiertes Phenyl steht,

X für oder Chlor steht und

Y für Wasserstoff, Fluor oder Chlor steht, dadurch gekennzeichnet, daß man Ketone der Formeln

$$R^2-CH=CH-CO-\underset{CH_2Y}{\overset{CH_2X}{C}}-CH_3 \qquad \text{(IId)}$$

oder

$$R^2-C\equiv C-CO-\underset{CH_2Y}{\overset{CH_2X}{C}}-CH_3 \qquad \text{(IIe)}$$

in welchen $R^2$, X und Y die oben angegebenen Bedeutungen haben, mit Wasserstoff in Gegenwart eines Verdünnungsmittels und in Gegenwart eines Katalysators umsetzt.

8. Substituiertes 5-Cycloalkyl-2,2-dimethyl-pentan-3-on- der Formel

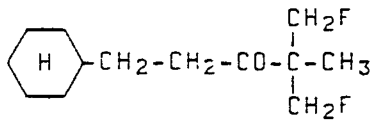

**Claims**

1. Substituted 5-cycloalkyl-2,2-dimethyl-pentan-3-ones of the formula

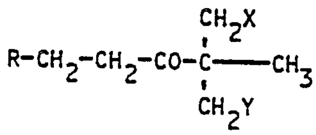

(I)

in which

R represents cycloalkyl which has 5 to 7 carbon atoms and is optionally mono- to trisubstituted by identical or different alkyl radicals with 1 to 3 carbon atoms,

X represents fluorine or chlorine and

Y represents hydrogen, fluorine or chlorine.

2. Process for the preparation of substituted 5-cyclo-alkyl-2,2-dimethyl-pentan-3-ones of the formula

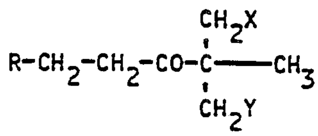

(I)

in which

R represents cycloalkyl which has 5 to 7 carbon atoms and is optionally mono to trisubstituted by identical or different alkyl radicals with 1 to 3 carbon atoms,

X represents fluorine or chlorine and

Y represents hydrogen, fluorine or chlorine,

characterised in that ketones of the formulae

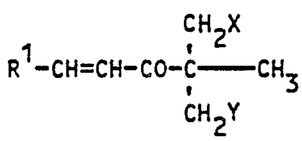

(IIa)

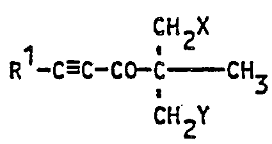

(IIb)

or

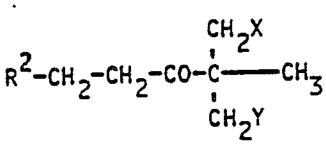

(IIc)

in which

$R^1$ represents cycloalkyl with 5 to 7 carbon atoms, cycloalkenyl with 5 to 7 carbon atoms or phenyl, in each case optionally mono- to trisubstituted by identical or different alkyl radicals with 1 to 3 carbon atoms,

$R^2$ represents phenyl which is optionally mono- to trisubstituted by identical or different alkyl radicals with 1 to 3 carbon atoms and

X and Y have the abovementioned meanings,

are reacted selectively with hydrogen in the presence of a hydrogenation catalyst and if appropriate in the presence of a diluent.

3. Process for the preparation of 4-azolyl-5-cycloalkyl-2,2-dimethyl-pentan-3-ones and -ols of the formula

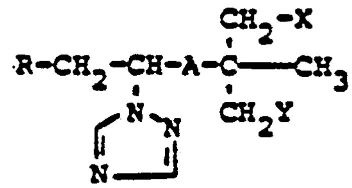

(VII)

in which

R represents cycloalkyl which has 5 to 7 carbon atoms and is optionally mono- to trisubstituted by identical or different alkyl radicals with 1 to 3 carbon atoms,

X represents fluorine or chlorine and

Y represents hydrogen, fluorine or chlorine, and

A represents a keto or a —CH(OH) group,

characterised in that substituted 5-cycloalkyl-2,2-dimethyl-pentan-3-ones of the formula

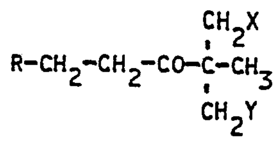

(I)

in which R, X and Y have the abovementioned meanings, are reacted with chlorine or bromine in the presence of an inert organic solvent, or with chlorinating agents, if appropriate in the presence of a diluent, then the halogenoketones thus obtained, of the formula

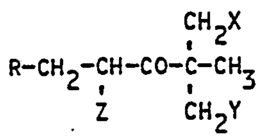

(VIII)

in which

R, X and Y have the abovementioned meanings and

Z represents chlorine or bromine,

are reacted with 1,2,4-triazole in the presence of an inert organic solvent, and then, if appropriate, the 4-azolyl-5-cycloalkyl-2,2-dimethylpentan-3-ones thus obtained, of the formula

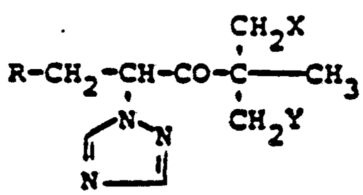

(VIIa)

in which R, X and Y have the abovementioned meanings, are reacted either with complex hydrides in the presence of a polar organic solvent or with aluminium isopropylate in the presence of a diluent.

4. Ketone of the formula

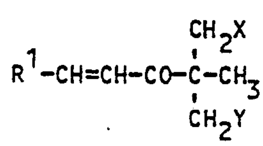

(IIa)

in which

$R^1$ represents cycloalkyl with 5 to 7 carbon atoms, cycloalkenyl with 5 to 7 carbon atoms or phenyl in each case optionally mono- to trisubstituted by identical or different alkyl radicals with 1 to 3 carbon atoms,

X represents fluorine or chlorine and

Y represents hydrogen, fluorine or chlorine.

5. Process for the preparation of ketones of the formula

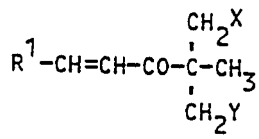

(IIa)

in which

$R^1$ represents cycloalkyl with 5 to 7 carbon atoms, cycloalkenyl with 5 to 7 carbon atoms or phenyl, in each case optionally mono- to trisubstituted by identical or different alkyl radicals with 1 to 3 carbon atoms,

X represents fluorine or chlorine and

Y represents hydrogen, fluorine or chlorine,

characterised in that butan-2-ones of the formula

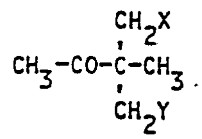

(III)

in which X and Y have the abovementioned meanings, are reacted with aldehydes of the formula

$$R^1—CH = O \quad (IV)$$

in which $R^1$ has the abovementioned meanings, in the presence of a diluent and in the presence of a base.

6. Ketones of the formula

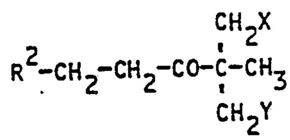

(IIc)

in which

$R^2$ represents phenyl which is optionally mono- to trisubstituted by identical or different alkyl radicals with 1 to 3 carbon atoms,

X represents fluorine or chlorine and

Y represents hydrogen, fluorine or chlorine.

7. Process for the preparation of ketones of the formula

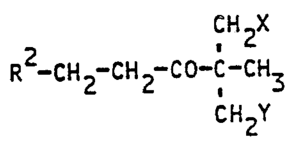

(IIc)

in which

$R^2$ represents phenyl which is optionally mono- to trisubstituted by identical or different alkyl radicals with 1 to 3 carbon atoms,

X represents fluorine or chlorine and

Y represents hydrogen, fluorine or chlorine,

characterised in that ketones of the formulae

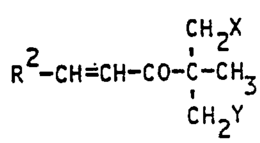

(IId)

or

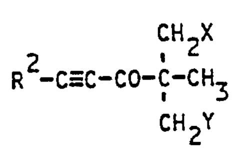

(IIe)

in which $R^2$, X and Y have the abovementioned meanings, are reacted with hydrogen in the presence of a diluent and in the presence of a catalyst.

8. Substituted 5-cycloalkyl-2,2-dimethyl-pentan-3-one of the formula

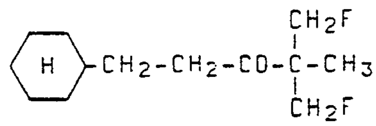

**Revendications**

1. 5-cycloalkyl-2,2-diméthyl-pentane-3-ones substituées de formule

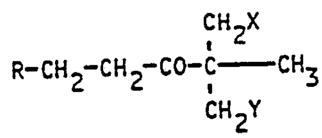

(I)

dans laquelle

R représente un groupe cycloalkyle en $C_5$-$C_7$ éventuellement mono- à tri-substitué par des groupes alkyle identiques ou différents en $C_1$-$C_3$,

X représente le fluor ou le chlore et

Y représente l'hydrogène, le fluor ou le chlore,

2. Procédé de préparation des 5-cycloalkyl-2,2-diméthyl-pentane-3-ones substituées de formule

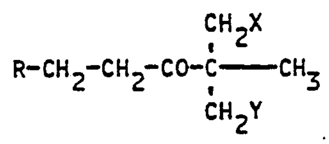

(I)

dans laquelle

R représente un groupe cycloalkyle en $C_5$-$C_7$ éventuellement mono- à tri-substitué par des groupes alkyle identiques ou différents en $C_1$-$C_3$,

X représente le fluore ou le chlore et

Y représente l'hydrogène, le fluor ou le chlore,

caractérisé en ce que l'on fait réagir sélectivement avec l'hydrogène, en présence d'un catalyseur d'hydrogénation et le cas échéant en présence d'un diluant, des cétones de formules

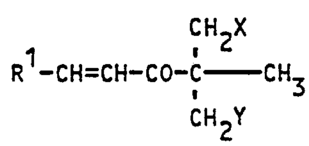

(IIa)

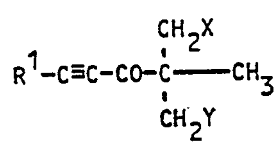

(IIb)

ou

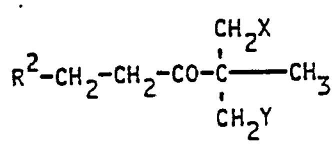

(IIc)

dans lesquelles

$R^1$ représente un groupe cycloalkyle en $C_5$-$C_7$, cycloalcényle en $C_5$-$C_7$ ou phényle, chacun éventuellement mono- à tri-substitué par des groupes alkyle identiques ou différents en $C_1$-$C_3$,

$R^2$ représente un groupe phényle éventuellement mono- à tri-substitué par des groupes alkyle identiques ou différents en $C_1$-$C_3$ et

X et Y ont les significations indiquées ci-dessus.

3. Procédé de préparation des 4-azolyl-5-cycloalkyl-2,2-diméthyl-pentane-3-ones et -ols de formule

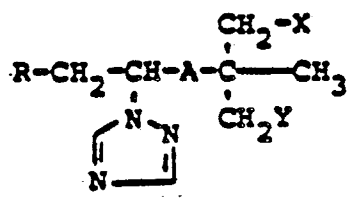

(VII)

dans laquelle

R représente un groupe cycloalkyle en $C_5$-$C_7$ éventuellement mono- à tri-substitué par des groupes alkyle identiques ou différents en $C_1$-$C_3$,

X représente le fluor ou le chlor et

Y représente l'hydrogène, le fluor ou le chlore,

A représente un groupe céto ou un gorupe —CH(OH),

caractérisé en ce que l'on fait réagir les 5-cycloalkyl-2,2-diméthyl-pentane-3-ones substituées de formule

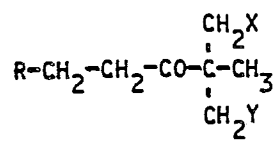

(I)

dans laquelle R, X et Y ont les significations indiquées ci-dessus, avec le chlore ou le brome en présence d'un solvant organique inerte, ou avec des agents chlorants, éventuellement en présence d'un diluant, ce qui donne les halogénocétones de formule

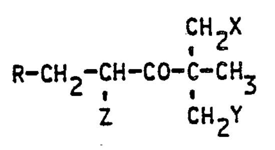

(VIII)

dans laquelle

R, X et Y ont les significations indiquées ci-dessus et

Z représente le chlore ou le brome,

qu'on fait réagir avec le 1,2,4-triazole en présence d'un solvant organique inerte, après quoi, le cas échéant, on fait réagir les 4-azolyl-5-cycloalkyl-2,2-diméthylpentane-3-ones obtenues, de formule

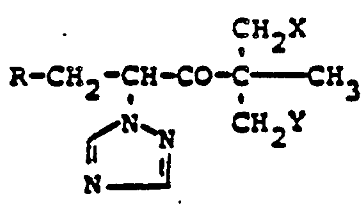

(VIIa)

dans laquelle R, X et Y ont les significations indiquées ci-dessus, soit avec des hydrures complexes en présence d'un solvant organique polaire, soit avec l'isopropylate d'aluminium en présence d'un diluant.

4. Cétones de formule

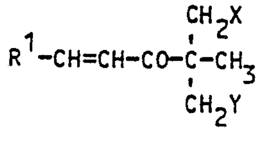

(IIa)

dans laquelle

$R^1$ représente un groupe cycloalkyle en $C_5$-$C_7$, cycloalcényle en $C_5$-$C_7$ ou phényle, chacun éventuellement mono- à tri-substitué par des groupes alkyle identiques ou différents en $C_1$-$C_3$,

X représente le fluor ou le chlore et

Y représente l'hydrogène, le fluor ou le chlore.

5. Procédé de préparation des cétones de formule

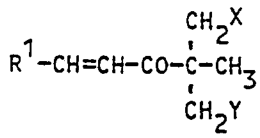

(IIa)

dans laquelle

$R^1$ représente un groupe cycloalkyle en $C_5$-$C_7$, cycloalcényle en $C_5$-$C_7$ ou phényle, chacun éventuellement mono- à tri-substitué par des groupes alkyle identiques ou différents en $C_1$-$C_3$,

X représente le fluor ou le chlore et

Y représente l'hydrogène, le fluor ou le chlore,

caractérisé en ce que l'on fait réagir des butane-2-ones de formule

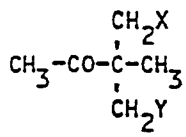

(III)

dans laquelle X et Y ont les significations indiquées ci-dessus, avec des aldéhydes de formule

$$R^1—CH = O \qquad (IV)$$

dans laquelle $R^1$ a les significations indiquées ci-dessus, en présence d'un diluant et en présence d'une base.

6. Cétones de formule

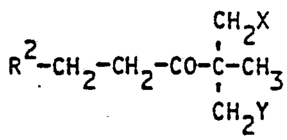

(IIc)

dans laquelle

$R^2$ représente un groupe phényle éventuellement mono- à tri-substitué par des groupes alkyle identiques ou différents en $C_1$-$C_3$,

X représente le fluor ou le chlore et

Y représente l'hydrogène, le fluor ou le chlore.

7. Procédé de préparation des cétones de formule

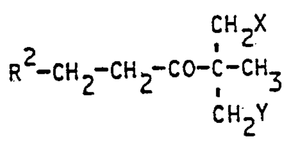

(IIc)

dans laquelle

$R^2$ représente un groupe phényle éventuellement mono- à tri-substitué par des groupes alkyle identiques ou différents en $C_1$-$C_3$,

X représente le fluor ou le chlore et

Y représente l'hydrogène, le fluor ou le chlore,

caractérisé en ce que l'on fait réagir avec l'hydrogène, en présence d'un diluant et en présence d'un catalyseur, des cétones de formules

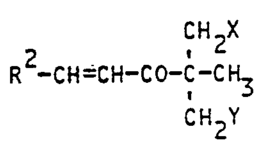

(IId)

ou

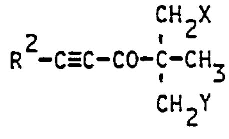

(IIe)

dans lesquelles $R^2$, X et Y ont les significations indiquées ci-dessus.

8. La 5-cycloalkyl-2,2-diméthyl-pentane-3-one substituée de formule